# EUROPÄISCHE PATENTSCHRIFT

(11) **EP 1 028 933 B1**
(45) Veröffentlichungstag und Bekanntmachung des Hinweises auf die Patenterteilung: **19.12.2001**
(21) Anmeldenummer: 98956881.1
(22) Anmeldetag: 28.10.1998
(51) Int. Cl.: C05G 3/00

(54) **FLIESSFÄHIGE MEHRKOMPONENTENGEMISCHE ZUR WACHSTUMSFÖRDERUNG DER ERDREICH-MIKROORGANISMENFLORA UND IHRER ANWENDUNG**
FLOWABLE MULTICOMPONENT MIXTURES FOR PROMOTING THE GROWTH OF THE SOIL MICRO-ORGANISM FLORA, AND USE OF THE SAME
MELANGES MULTICONSTITUANTS COULANTS POUR STIMULER LA CROISSANCE DE LA FLORE MICROBIENNE DU SOL, ET LEUR UTILISATION

(30) Priorität: 06.11.1997 DE 19748884
(43) Veröffentlichungstag der Anmeldung: 23.08.2000
(73) Patentinhaber: Cognis Deutschland GmbH, 40589 Düsseldorf (DE)
(72) Erfinder: VON TAPAVICZA, Stephan, D-40699 Erkrath (DE); KOPP-HOLTWIESCHE, Bettina, D-40599 Düsseldorf (DE); BELL, Doris, D-40215 Düsseldorf (DE); BÖTTCHER, Heinz, D-40699 Erkrath (DE); MÜNTEFERING, Hubert, D-59590 Geseke (DE)
(86) Internationale Anmeldenummer: EP9806823
(87) Internationale Veröffentlichungsnummer: WO9924380

(56) Entgegenhaltungen:
- DE-A- 4 437 313
- DE-A- 19 701 127
- US-A- 5 482 529

## Beschreibung

Das in der jüngeren Vergangenheit entwickelte Fachwissen zu den scheinbar so einfachen Vorgängen des Erdreichaufschlusses zur Förderung des Pflanzenwachstums und Zusatz von Hilfsstoffen aus dem Bereich der Düngemittel läßt Einblicke in die hochkomplexen Lebens- und Austauschvorgänge zu, die in diesem Bereich des lebenden Erdreichsubstrats stattfinden. Eine zusammenfassende Darstellung und Aufgliederung dieses Bereiches natürlicher Lebensvorgänge findet sich beispielsweise in der in Buchform erschienen Veröffentlichung "Soil Microbiology and Biochemistry", Verfasser E.A. Paul und F.E. Clark, erschienen in Academic Press, Inc., San Diego, USA, 1989. Zusammenfassend dargestellt sind hier insbesondere die vielgestaltigen und außerordentlich komplexen Lebensvorgänge und Interaktionen der vielgestaltigen Mikroorganismenflora und der damit im Austausch stehenden Mikro- und Makro-Bodenfauna des belebten Erdreiches, im nachfolgenden auch mit Substrat bezeichnet. Die lebende Pflanze steht über ihren Wurzelbereich im kontinuierlichen Austausch mit diesen Lebensvorgängen der Erdreich-Mikroorganismen. Der Steuerung und Pflege dieses Mikroorganismenwachstums und dabei primär des Wachstums der Mikroorganismenflora im Erdreich kommt damit entscheidende Bedeutung für die Pflege und Steuerung des Pflanzenwachstums zu. Die Konzentration der Mikroorganismenflora ist in dem im unmittelbaren Kontakt mit der Pflanzenwurzel stehenden Erdbodenbereich - in der Fachsprache als Rhizosphere bezeichnet - am höchsten und fällt im Abstandsbereich weniger Millimeter zunehmend und stark ab. Dem Stoffaustausch zwischen Pflanzenwurzel und Mikroorganismenpopulation im Rhizospherenbereich kommt damit entscheidende Bedeutung für die Steuerung, Pflege und Förderung des Pflanzenwachstums zu. Mangelerscheinungen - z.B. bezüglich des Zutritts von Sauerstoff, Wasser und darin gelösten Nährstoffen - können die Pflanzen schwächen und damit schwerwiegende Störungen auslösen. Diese Störungen können sowohl im Wurzelbereich - z.B. Nematodenbefall - als auch im oberirdischen Pflanzenbereich auftreten. Die im nachfolgenden geschilderte technische Lehre der Erfindung geht von der Konzeption aus, eine Optimierung und natürliche Steuerung und Förderung der Mikroorganismenprozesse im Substrat und dabei insbesondere der primären Förderung der Wachstumsprozesse der Mikroorganismenflora sicherzustellen. Die Steigerung der mikrobiellen Aktivität im Boden fördert natürlich stattfindende Mineralisierungsprozesse im Sinne des Bodenaufschlusses und Rückführung abgestorbener Pflanzenteile in den Nährstoffkreislauf. Die im Wachstum geförderten Mikroorganismenpopulationen dienen als Nahrung vieler Kleinstlebewesen, wodurch letztlich eine allgemeine Belebung des Bodens erfolgt.

Die verbesserte Bodenstruktur erlaubt eine optimale Sauerstoffversorgung von Boden und Pflanzenwurzel, die Förderung der mikrobiellen Aktivität bewirkt eine Stabilisierung der Bodenmatrix. Die verbesserte Durchlüftung zeigt eine rottebeschleunigende Wirkung und sorgt damit für einen lockeren Boden, der auch für den Stoffwechsel der Pflanzenwurzel bedeutsam ist. Bodenverdichtungen aufgrund hoher Niederschläge oder unsachgemäßer Bearbeitung können verhindert oder wenigstens herabgesetzt werden.

Die DE 44 37 313 beschreibt die Verwendung ausgewählter, Phosphor und Stickstoff enthaltender Komponenten, aus der Klasse der Phospholipide zur Verbesserung des Pflanzenwachstums. Durch Zusatz dieser Phospholipide zum Substrat, auf dem die Pflanzen wachsen oder wachsen sollen, läßt sich das Wachstum dieser Pflanzen verbessern. Nach den Angaben dieser Druckschrift wird vermutet, daß diese Wachstumssteigerung mit einer Stimulierung der im Substrat lebenden Mikroorganismen zusammenhängt. Als Phospholipide kommen in erster Linie Lecithin, Lecithinhydrolysate und chemisch modifizierte Lecithine in Betracht. Schon die DE 42 18 243 beschreibt Nährstoffgemische auf Basis flüssiger wäßriger Zubereitungen, die einen Ester der Phosphorsäure als Emulgator und P-Quelle sowie gewünschtenfalls eine oder mehrere wasserlösliche oder wasserdispergierbare N-Quellen enthalten. Zielvorstellung ist hier die An- und Aufzucht von Mikroorganismen, die - im Rahmen einer Bioremediationzum biologischen Abbau von Mineralölverschmutzungen in entsprechend belasteten Erdbodenbereichen und/oder in Gewässern geeignet sind.

Die WO 93/01150 beschreibt einfach zu handhabende Düngemittelgemische zum Eintrag von N in das Pflanzenwachstum. Vorgesehen wird hier zusammen mit den Abmischungen von Düngemitteln auf Basis von Makro- und Mikronährstoffen für die Pflanzenaufzucht, Wasser und eine Ölphase in Gegenwart von W/O-Invertemulgatoren einzusetzen. Sichergestellt werden soll auf diese Weise die Ausbildung von insbesondere pastenförmigen Abmischungen, in denen die geschlossene Ölphase in Filmform die wäßrigen Mischungsanteile trennen bzw. umhüllen soll. Als gleichwertige Ölphasen sind Öle pflanzlichen Ursprungs und Mineralöl aufgeführt.

Gegenstand der nicht vorveröffentlichten Deutschen Patentanmeldung P19701127.6 ist eine schaumarme Netzhilfe in der Angebotsform eines hochkonzentrierten, gleichwohl fließ- und gießfähigen wäßrigen Konzentrats auf Tensidbasis zur Intensivierung des Eindringens und Spreitens von Wasser im Bereich der Pflanzenverwurzelung bei deren Bewässerung, enthaltend als ökologisch verträgliche Tensidkomponente Alkyl(poly)glykosidverbindungen vom O/W-Typ (APG-Verbindungen), olefinisch ungesättigte Alkohole als Schaumbremse/Entschäumer und niedere wasserlösliche Alkohole als Viskositätsregler. Die im Zusammenhang mit dem Eintrag dieser Netzhilfe in der Praxis beobachtete Verbesserung des Pflanzenwachstums an Trockenstellen im Rasenwachstums, die insbesondere durch den sogenannten Rasenfilz befallen sind, wird auf die bessere Spreitung des Wassers als lebensfördernde Komponente im Rasenwurzelbereich zurückgeführt.

Die im nachfolgenden geschilderte neue technische Lehre zur Förderung und Pflege des Pflanzenwachstums durch Steuerung der natürlichen Wachstumsprozesse im Substrat geht von der Konzeption aus, primär eine Förderung, Steuerung und Sicherstellung des Mikroorganismenwachstums durch Eintrag eines im nachfolgenden geschilderten Mehrkomponentengemisches zu bewirken. Diese primäre Förderung des Mikroorganismenwachstums soll dabei insbesondere auch gerade im Rhizospherenbereich und damit in dem für das Pflanzenwachstum entscheidenden Bereich des mit den Pflanzenwurzeln durchsetzten Substrats sichergestellt werden. Die erfindungsgemäße Lehre wird dabei durch zwei übergeordnete Konzeptionen gelenkt: Zusammen mit Phosphor (P) und Stickstoff (N) enthaltenden Trägerstoffen und gewünschtenfalls weiteren Pflanzen-, Makro- und/oder Mikronährstoffen sollen jetzt ausgewählte Kohlenwasserstoffreste enthaltende Verbindungen als zusätzliche C-Lieferanten für das Wachstum der Mikroorganismenflora in den Boden eingetragen werden. Gleichzeitig soll durch die Zubereitung dieser Wachstumshilfsstoffe und ihre Anwendungsform deren optimierte Spreitung einschließlich des Eintrages in den Rhizospherenbereich des Substrats ermöglicht werden.

### Gegenstand der Erfindung

Gegenstand der Erfindung ist dementsprechend in einer ersten Ausführungsform ein Verfahren zur Wachstumssteigerung der Erdreich-Mikroorganismenflora und Förderung des mikrobiellen Aufschlusses eines mit organischer Substanz beladenen Erdreichs (Substrat) sowie damit verbundener nachhaltiger Stärkung der Pflanzengesundheit und Förderung eines gesunden Pflanzenwachstums in diesem Substrat. Die erfindungsgemäße Lehre ist hier gekennzeichnet durch den Eintrag wäßriger Zubereitungen, enthaltend
(a) ökologisch verträgliche Netzmittel vom O/W-Typ zusammen mit
(b) lipophile gesättigte und/oder olefinisch ungesättigte Kohlenwasserstoffreste mit Fettstruktur aufweisenden und sowohl aerob als auch anaerob abbaubaren organischen Verbindungen als zusätzliche C-Lieferanten für das Wachstum der Mikroorganismenflora,
verbunden mit gleichzeitigem und/oder zeitlich versetzten Eintrag von
(c) wenigstens anteilig lipophile Reste aufweisende und dabei bevorzugt Öl-lösliche Verbindungen des P und/oder N sowie gewünschtenfalls weitere Makro- und/oder Mikronährstoffe für das Pflanzenwachstum enthaltenden Trägerstoffen.

Die Erfindung betrifft in einer weiteren Ausführungsform wasserverdünnbare wäßrige Konzentrate von Hilfsstoffen für die Wachstumssteigerung der das Pflanzenwachstum fördernden Erdreich-Mikroorganismenflora, enthaltend die zuvor genannten Komponenten zu (c) und (a), wobei das Kennzeichen darin liegt, daß diese Mehrstoffgemische zusätzlich feinstteilig emulgierbare und/oder dispergierbare C-Lieferanten für das Wachstum der Mikroorganismenflora aus der zuvor genannten Wertstoffkiasse zu (b) enthält.

Erfindungsgegenstand ist schließlich die Anwendung des Arbeitsprinzips einer gesunden Wachstumssteigerung der Erdboden-Mikroorganismenflora und der damit verbundenen Anregung und Entwicklung der Erdbodenfauna durch Eintrag der zuvor genannten Mehrkomponentengemische für eine Erdbodenkur mit rottebeschleunigender Wirkung (Mineralisierung), mit bodenverbessernder Wirkung (Verbesserung der Bodenbelüftung, Beseitigung von anaeroben Bodenbereichen) und insbesondere für den Einsatz als Vitalisierungsmittel mit pflanzenstärkender Düngewirkung, die bevorzugt als slow-release-Düngewirkung ausgelegt ist.

### Einzelheiten zur erfindungsgemäßen Lehre

Bevor auf die nähere Definition der die erfindungsgemäße Lehre bestimmenden Einzelelemente eingegangen wird, seien im nachfolgenden - ohne Anspruch auf Vollständigkeit - einige Überlegungen zum Arbeitsprinzip der neuen technischen Lehre und der damit verknüpften vielgestaltigen synergistischen Effekte zur Ergebnisoptimierung zusammengefaßt.

Die Offenbarung der eingangs zitierten Druckschrift DE 44 37 313 will das Pflanzenwachstum in Erdreichsubstraten durch Zusatz von Phospholipiden zum Substrat verbessern. Dabei geht diese Lehre von der Feststellung aus, daß durch Zusatz dieser P und N sowie lipophile Reste enthaltenden Hilfsstoffe auch die in nicht kontaminierten Böden natürlicherweise lebenden Mikroorganismen stimmuliert werden können. Das hat zur Folge, daß die auf den so behandelten Böden wachsenden Pflanzen besser gedeihen. Die erfindungsgemäße Lehre macht von diesem Arbeitsprinzip Gebrauch, geht aber jetzt einen entscheidenden Schritt darüber hinaus: Zusätzlich zu den P, N und gewünschtenfalls weitere Makro- und/oder Mikronährstoffe für das Pflanzenwachstum enthaltenden Trägerstoffen werden als zusätzliches Stimulanz für das Wachstum der Mikroorganismenflora ausgewählte C-Lieferanten in das Substrat eingetragen. Bei diesen erfindungsgemäß jetzt vorgesehenen zusätzlichen Kohlenstofflieferanten für das Mikroorganismenwachstum handelt es sich um lipophile gesättigte und/oder olefinisch ungesättigte Kohlenwasserstoffreste mit Fettstruktur aufweisende und sowohl aerob als auch anaerob abbaubare organische Verbindungen. Durch Eintrag dieser organisch gebundenen Kohlenstoff mit einer Vielzahl der energiereichen C-H-Bindungen enthaltenden Komponenten wird primär das Wachstum organotropher Mikroorganismen gefördert. Bekanntlich zählt die Mehrzahl der heute bekannten Bakterien-Spezies zu dieser Klasse organotropher Organismen. Ihr gesteigertes Wachstum führt dann in nachfolgenden komplexen Sekundärschritten zur Belebung der Bodenfauna. Die synergistische Wirkungssteigerung ist damit aber nur bruchstückweise erfaßt:

Eine wesentliche weitere Komponente der erfindungsgemäß vorgesehenen Mehrstoffgemische sind die ökologisch verträglichen Netzmittel vom O/W-Typ, die sich als Bestandteil des wäßrigen Zusatzes zum Erdreichsubstrat durch eine ausgesprochene Multifunktionalität auszeichnen. Die Spreitung der auf das Substrat aufgebrachten wäßrigen Phase, insbesondere im Bereich der Pflanzenwurzel, wird optimiert. Wasser kommt damit in den unmittelbaren Kontaktbereich der Wurzelhaare, wesentlich für die Wasser- und Nährstoffaufnahme. Verbunden damit ist gleichzeitig aber auch die gegebenenfalls wünschenswerte Reinigung der Porenstruktur, beispielsweise durch Freiwaschen von Substratbereichen mit unzureichendem Gasphasenaustausch unter Ausbildung einer hier nicht erwünschten anaeroben Mikroorganismen-Population. Die Mitverwendung dieser Netzhilfe führt gleichzeitig zur homogen Spreitung der das Mikroorganismenwachstum fördernden Hilfsstoffe und insbesondere der hier erfindungsgemäß vorgesehenen C-Lieferanten für das Organotrophen-Wachstum.

Die synergistische Wertstoffergänzung macht sich aber auch schon in Arbeitsschritten vor und beim Eintrag der erfindungsgemäßen Wachstumshilfen bemerkbar: Tensidische Netzhilfen und insbesondere die erfindungsgemäß bevorzugten Netzhilfen vom APG-Typ zeichnen sich in ihren wäßrigen Zubereitungen durch ein besonders ausgeprägtes Schaumbildungsvermögen aus. Es ist einleuchtend, daß für die praktische Handhabbarkeit der Hilfsmittel im land- und forstwirtschaftlichen Bereich eine solche Schaumbildung als ausgesprochen nachteilig empfunden wird. Hier hat sich nun aber wieder gezeigt, daß durch die gemeinsame Verwendung der erfindungsgemäß definierten Netzmittel zur Komponente (a) und der Kohlenwasserstoffreste mit Fettstruktur aufweisenden C-Lieferanten für das Wachstum der Mikroorganismenflora zu (b) derart ausgeprägte schaumdämpfende bzw. schauminhibierende Effekte eingestellt werden können, daß für das praktische Arbeiten mit den erfindungsgemäß definierten Wertstoffgemischen in wäßrigen Zubereitungen keine Probleme der hier angesprochenen Art bestehen.

Schließlich sei ein weiterer aus heutiger und zukünftiger Sicht besonders wichtiger Aspekt der erfindungsgemäßen Lehre angesprochen: Die Bewertung technologischer Neu- und Weiterentwicklungen wird schon heute sehr stark durch die Begriffe der "Sustainability" bzw. "Sustainable Development" bestimmt. Ganz besonders gilt diese Zusatzanforderung für den Bereich agro-biologischer und agro-chemischer Prozesse der durch die erfindungsgemäße Lehre angesprochenen Art. Die im nachfolgenden im einzelnen geschilderten Elemente der erfindungsgemäßen Lehre ermöglichen hier eine Optimierung, ohne dabei den Gesichtspunkt der Wirtschaftlichkeit außer Acht lassen zu müssen. Praktisch sämtliche Komponenten des erfindungsgemäß zum Einsatz kommenden Nährstoffsystems können als Naturstoff-basierte Chemikalien ausgestaltet sein. Die Quelle für alle diese Komponenten ist das gesunde Pflanzenwachstum. Es leuchtet sofort ein, daß hier gerade für den natürlichen Kohlenstoffkreislauf nicht nur zusätzliche Belastungen ausgeschlossen, sondern positive Eingriffe ermöglicht werden, die helfen können heute bereits bestehende Schäden zu mildern und abzubauen.

Nachfolgend werden zunächst detaillierte Angaben zu den erfindungsgemäß definierten Wertstoffkomponenten zu (a), (b) und (c) gemacht.

### Zu (a) "ökologisch verträgliche Netzmittel vom O/W-Typ"

Die hier angesprochenen Netzmittel bzw. Tenside ordnen sich insbesondere den Klassen anionischer Tenside und/oder nichtionischer Tenside zu. Eine wichtige Voraussetzung ist ihre ökologische Verträglichkeit und damit insbesondere eine hinreichende biologische Abbaubarkeit im Substrat. Biologisch schnell und vollständig abbaubare Tensidverbindungen aus der Klasse nichtionischer Tenside sind eine bevorzugte Stoffklasse der hier angesprochenen Hilfsstoffe.

Geeignete anionische Tenside sind beispielsweise Seifen, aber auch biologisch abbaubare Alkylsulfate, insbesondere Fettalkoholsulfate. Schwer bzw. nur unvollständig abbaubare Tenside auf petrochemischer Basis, beispielsweise Alkylbenzolsulfonat oder Alkylethersulfate sind weniger geeignet. Geeignete Vertreter können sein die Partialester der Phosphorsäure mit Fettalkoholen und dabei insbesondere entsprechende Partialester mit geradkettigen Fettalkoholen, bevorzugt natürlichen Ursprungs und damit gerader Kohlenstoffzahl. Geeignet sind hier beispielsweise entsprechende Ester kürzerkettiger Fettalkohole, etwa solcher mit 6 bis 10 C-Atomen im Fettalkoholmolekül. Aber auch Alkylphosphate mit längeren Fettalkoholresten mit beispielsweise 12 bis 24 C-Atomen sind grundsätzlich geeignet. Entsprechendes gilt - wenn auch weniger bevorzugt - für die vergleichbaren Fettalkoholetherphosphate.

Erfindungsgemäß besonders bevorzugte biologisch abbaubare Tenside zur Stoffklasse (a) sind entsprechende Verbindungen wenigstens überwiegend nichtionischen Charakters, die weiterhin bevorzugt zum wenigstens überwiegenden Anteil Naturstoff-basierten Ursprungs sind und dabei bevorzugte HLB-Werte im Bereich von 10 bis 18 aufweisen.

Erfindungsgemäß ist es besonders bevorzugt, als Komponente (a) wenigstens anteilsweise und dabei insbesondere wenigstens überwiegend Alkyl(oligo)glukosid-Verbindungen einzusetzen, deren Alkylrest sich wenigstens überwiegend von geradkettigen Fettalkoholen ableitet. Verbindungen dieser Art - nach heutigem Sprachgebrauch auch als APG-Komponenten bzw. -Verbindungen bezeichnet - sind tensidische Hilfsstoffe eines breiten Einsatzbereiches. Für ihren heute im großtechnischen Maßstab stattfindenden Einsatz in der Praxis sind eine Mehrzahl von Faktoren wichtig: Netzmittel auf APG-Basis können bekanntlich voll Naturstoff-basiert sein. Sie fallen als Reaktionsprodukte durch Umsetzung von Fettalkoholen mit Glukose, Oligoglukosen oder auch - bei gleichzeitigem Abbau der Kettenlänge -mit Polyglykosiden wie Stärke als Reaktionsprodukte der allgemeinen Formel R-O-(G)ₓ an, in der R einen primären, bevorzugt geradkettigen und aliphatischen Kohlenwasserstoffrest mit wenigstens 6 C-Atomen, vorzugsweise mit 8 bis 24 C-Atomen und insbesondere 8 bis 18 C-Atomen, bedeutet und G für eine Glykoseeinheit mit 5 oder 6 C-Atomen, vorzugsweise für Glukose, steht. Der Oligomerisierungsgrad x - und damit der DP-Wert - der die Verteilung von Monoglykosiden und Oligoglykosiden angibt, ist in der hier betroffenen Tensidklasse üblicherweise ein Wert zwischen 1 und 10 und liegt beispielsweise im Bereich von etwa 1,2 bis 5, vorzugsweise im Bereich von etwa 1,2 bis 4 und insbesondere im Bereich von 1,2 bis 2. Auf das umfangreiche Fachwissen und Schrifttum zur Herstellung und Beschaffenheit von APG-Verbindungen der hier betroffenen Art kann verwiesen werden, siehe beispielsweise die in Buchform erschienene Veröffentlichung von Hill et. al. "Alkyl Polyglykosides", VCH-Verlagsgesellschaft mbH, Weinheim, 1997.

Wenn auch die hier angesprochene Klasse der APG-Verbindungen vom O/W-Typ die bevorzugten Vertreter für die erfindungsgemäß einzusetzenden Netzmittel der Klasse (a) sind, so gilt gleichwohl, daß auch andere biologisch abbaubare und verträgliche Komponenten mit vergleichbaren Eigenschaften im Sinne der erfindungsgemäßen Lehre verwendet bzw. mitverwendet werden können. Beispiele sind hier Zuckerpartialester von Monocarbonsäuren mit insbesondere 8 bis 24 C-Atomen, Sorbitanester, etwa von der Art des Sorbitanmonostearat oder Sorbitanmonoleat oder aber auch Tenside biologischen Ursprungs. Als Beispiele seien hier benannt Sophorose-Lipid, Trehalose-Lipid oder Lipopeptide, wie sie als Stoffwechselprodukte oder Membranbestandteile einer Mehrzahl von Mikroorganismenstämmen bekannt sind.

### C-Lieferanten für das Wachstum der Mikroorganismenflora gem. (b)

Wie zuvor ausgeführt, kommt im Rahmen der erfindungsgemäßen Lehre der Mitverwendung dieser Komponente(n) nach Art und Menge gegenüber dem nächststehenden Stand der Technik - wie er sich beispielsweise aus der DE 44 37 313 ergibt - entscheidende Bedeutung zu. Dabei steht hier die Wachstumsförderung organotropher Mikroorganismen unter Einsatz dieser Komponente (b) als Kohlenstofflieferant für das Mikroorganismenwachstum im Vordergrund.

Ein wichtiges Charakteristikum für diese Zusatzkomponenten (b) ist der Bestimmungsparameter, daß sie durch natürliche Abbauprozesse sowohl aerob als auch anaerob abbaubar sind. Die für das organotrophe Wachstum erfindungsgemäß wesentliche C-Quelle sind die in dieser Komponente vorliegenden lipophilen Kohlenwasserstoffreste mit Fettstruktur und damit die vergleichsweise erhöhte Konzentration der energieliefernden C-H-Gruppierungen. Wie schon zuvor ausgeführt, können diese Kohlenwasserstoffreste mit Fettstruktur gesättigt und/oder auch wenigstens anteilsweise olefinisch ungesättigt sein. Weiterführende Überlegungen zur physikalisch-chemischen Beschaffenheit dieser Komponente, auf die im Nachfolgenden noch eingegangen wird, können hier mitbestimmend sein.

Bevorzugte Komponenten zu (b) sind Öl-lösliche, dabei jedoch biologisch verträgliche organische Verbindungen mit Fettresten der angegebenen Art, die wenigstens 6 C-Atome und insbesondere wenigstens 8 C-Atome, aufweisen. Dabei ist der Einsatz entsprechender Komponenten auf Basis geradkettiger Kohlenwasserstoffreste bzw. KW-verbindungen bevorzugt. Insbesondere Bedeutung haben entsprechende Komponenten, die wenigstens überwiegend Naturstoff-basiert sind.

Besonders wichtige Vertreter der hier angesprochenen Stoffklasse zu (b) sind entsprechende Kohlenwasserstoffverbindungen, die wenigstens anteilsweise mit Sauerstoff als Heteroatom funktionalisiert sind. Typische Beispiele für Komponenten dieser Art sind Fettalkohole und/oder Fettsäuren bzw. ihre Derivate und/oder Salze. Geeignete Fettalkohol- bzw. Fettsäurederivate sind deren Ester, Ether und/oder Amide. Besondere Bedeutung kommt im Rahmen der Erfindung den Fettalkoholen und den Estern von Fettsäuren mit einfunktionellen und/oder mehrfunktionellen Alkoholen zu. Der Begriff der Fettsäureester umfaßt dabei beim Einsatz mehrfunktioneller Alkohole sowohl die Vollester wie die Partialester. Welche speziellen Komponenten im jeweilig konkreten Einzelfall die bevorzugten Vertreter sind, wird gegebenenfalls durch Sekundäreffekte und damit durch das Vorliegen von gegebenenfalls gewünschten Synergismen innerhalb des Gesamtsystems bestimmt. Lediglich beispielhaft sei hier auf entsprechende Aussagen der älteren jedoch nicht vorveröffentlichten Patentanmeldung P 19701127.6 eingegangen:

Tensidbasierte wäßrige Zubereitungen und insbesondere entsprechende wäßrige APG-basierte Netzhilfsmittel zeichnen sich in der Regel durch das hohe Schaumvermögen dieser niotensidischen Hilfsmittel auf APG-Basis aus. Für das erfindungsgemäß betroffene Arbeitsgebiet kann das eine ausgesprochene Belastung darstellen. Hier stellt sich die zusätzliche Aufgabe durch Mitverwendung sogenannter Schaumbremsen bzw. Entschäumer Abhilfe zu schaffen. Fettalkohole, Partialester von insbesondere niederen mehrfunktionellen Alkoholen - z.B. Glycerin - und Fettsäuren und insbesondere ihre Abmischungen erfüllen diese Aufgabe. Gleichzeitig sind sie aber die erfindungsgemäß gewünschten C-Lieferanten für die Anregung und Steigerung des Mikroorganismenwachstums im Boden und damit optimale Vertreter für die Komponenten zu (b) im Sinne der erfindungsgemäßen Definition.

Die Abmischung wäßriger APG-Konzentrate mit Entschäumern/Schaumbremsen auf Alkoholbasis und/oder Basis von Partialestern von Fettsäuren und mehrwertigen Alkoholen, insbesondere Glycerin, kann allerdings zur Ausbildung nicht mehr fließfähig eingedickter Gele führen. Durch Zusatz begrenzter Mengen niederer mono- und/oder mehrfunktioneller Alkohole, z.B. durch Zusatz begrenzter Mengen an Ethanol zum gelförmig verdickten Wertstoffkonzentrat, wird es dann allerdings möglich auch im Bereich der Raumtemperatur die Fließ- und Gießfähigkeit wieder sicherzustellen.

Die im konkreten Einzelfall als Mischungskomponente (b) einzusetzenden Wertstoffe bzw. Wertstoffgemische werden somit in bevorzugten Ausführungsformen nicht nur durch Überlegungen zur Optimierung dieser Komponente als Kohlenstofflieferant für das Mikroorganismenwachstum bestimmt. Mitentscheidend können Sekundäreffekte wie Schaumarmut des wäßrigen Mehrkomponentengemisches, Homogenisierung des lipophile Komponenten zusammen mit Netzmitteln vom O/W-Typ in wäßriger Phase enthaltenden Mehrkomponentengemisches und Applizierbarkeit im Sinne der Verdünnung mit weiterem Wasser und anschließendem Ausbringen durch Gießen und/oder Sprühen sein. Die zuvor benannte ältere Anmeldung P 19701127.6 beschäftigt sich insbesondere mit diesen Aspekten. Zum Zwecke der Vervollständigung der Erfindungsoffenbarung wird der Gegenstand dieser älteren Anmeldung hiermit ausdrücklich auch zum Gegenstand der vorliegenden Erfindungsoffenbarung gemacht.

Insbesondere für den störungsfreien Eintrag der wasserbasierten Stoffgemische in das Erdreichsubstrat und den Transport der Kohlenstofflieferanten zur Mischungskomponente (b) im Sinne der erfindungsgemäßen Definition kann es wichtig sein, solche Komponenten zu (b) auszuwählen die wenigstens anteilsweise Stockpunkte gleich/kleiner 25 bis 30°C und insbesondere gleich/kleiner 10 bis 15°C, aufweisen. Geeignete Komponenten sind hier beispielsweise olefinisch ungesättigte C₁₂₋₂₄-Fettalkohole natürlichen Ursprungs, insbesondere wenigstens überwiegend C_{16/18}-Fettalkohole mit hohem Grad olefinischer Doppelbindungen und Erstarrungsbereichen gleich/kleiner 20°C, vorzugsweise gleich/kleiner 10 bis 15°C. Bevorzugte Mehrkomponentengemische zu diesem Bestandteil (b) im Sinne der erfindungsgemäßen Definition sind Abmischungen von Fettalkoholen mit Partialestern von gesättigten und insbesondere wenigstens anteilsweise olefinisch ungesättigten Fettsäuren mit mehrfunktionellen Alkoholen mit 2 bis 6 C-Atomen und insbesondere 3 bis 5 C-Atomen. So können insbesondere Glycerinpartialester von Fettsäuren natürlichen Ursprungs wichtige Mischungskomponenten für die Abmischung mit entsprechenden Fettalkoholen sein, wobei etwa gleiche Mengen an Fettalkohol und Fettsäurepartialester oder aber entsprechende Stoffgemische mit einem mehrfachen des Partialesters, bezogen auf den Fettalkohol, bevorzugte Stoffgemische sind. Geeignete Abmischungen von Fettalkohol zu Fettsäurepartialglycerid liegen beispielsweise im Bereich von etwa 1:1 bis 1:10, vorzugsweise 1:1 bis 1:5 und insbesondere von etwa 1:1 bis 1:3 Gewichtsteilen. Wie zuvor angegeben, können aber solche Fettsäurepartialester auch alleine als Komponente(n) zu (b) zum Einsatz kommen. Bevorzugt sind auch hier entsprechende Vertreter mit Stockpunkten in den zuvor genannten Bereichen.

Auf ein weiteres im erfindungsgemäßen Sinne wesentliches Bestimmungselement für das neue technische Handeln - nämlich auf die jeweils einzusetzenden Mindestmengen der C-Lieferanten für das Mikroorganismenwachstum im Rahmen der erfindungsgemäß insgesamt aufzubringenden Mehrkomponentengemische - wird im einzelnen zu einem späteren Zeitpunkt eingegangen. Hier sei nur vorab das Folgende klargestellt: Die erfindungsgemäße Lehre sieht als ein wesentliches Element die Mengenabstimmung der Komponente zu (b) auf die durch die Mischungskomponente zu (c) eingetragenen Mengen an P und gegebenenfalls weiteren Makro- und/oder Mikronährstoffen vor. Die Kohlenstoff für das Mikroorganismenwachstum liefernde Quelle zu (b) wird in solchen Mindestmengen eingesetzt, daß - bezogen auf den über die Mischungskomponente (c) eingetragenen Phosphor P - das Gewichtsverhältnis von C:P wenigstens im Bereich von etwa 5 bis 10:1 und vorzugsweise bei wenigstens etwa 20 bis 25:1 liegt. Je nach Bodenbeschaffenheit und dabei insbesondere je nach Art und Menge des im Bodenbereich vorliegenden organisch gebundenen Kohlenstoffs können allerdings Ausführungsformen bevorzugt sein, in denen wesentlich höhere C:P-Verhältnisse sichergestellt sind. So liegen wichtige untere Grenzwerte hier bei 40:1 und vorzugsweise im Bereich von wenigstens 50:1. Ein sehr viel größerer Überschuß des C-Lieferanten ist dabei in der Regel weiterhin möglich, so daß hier C:P-Gewichtsverhältnisse bis zu 500:1 oder auch noch darüber im Rahmen der erfindungsgemäßen Lehre liegen. Durch optimierte Spreitung dieses dem Mikroorganismen-Wachstum gut zugänglichen C-Lieferanten im Erdboden und durch seinen Transport bis in den Bereich der Rhizosphere wird damit die Anregung und Unterstützung des organotrophen Mikroorganismenwachstums im Sinne der erfindungsgemäßen Aufgabenstellung verwirklicht.

Die erfindungsgemäß eingesetzten Wertstoffe zu (b) mit ihren lipophilen Resten vom Fettcharakter und der aeroben als auch der anaeroben Abbaubarkeit sind vollständig zu CO₂, H₂O und Biomasse abbaubar. Als Ergebnis ist sichergestellt, daß sich bei ihrem Einsatz keine inerten oder ökotoxikologisch bedenklichen Abbauprodukte im Boden anreichern. Die lipophile Reste enthaltenden Komponenten zu (b) wandern im Boden nur langsam, sie tendieren dazu sich an lipophile bzw. oleophile Oberflächen und damit insbesondere auch an Wurzeloberflächen anzulagern. Sie werden praktisch nicht in das Grundwasser ausgewaschen und sind nicht toxisch, so daß ihre Anwendung auch aus diesem Grunde unbedenklich ist. Der zuletzt angesprochenen Interaktion zwischen den lipophilen Resten der erfindungsgemäß eingesetzten Mischungskomponenten zu (b) und weiteren Bestandteilen des Substrats bzw. des erfindungsgemäß zugesetzten Mehrkomponentengemisches kann besondere Bedeutung bei Auswahl geeigneter und optimierte Komponenten zur im nachfolgenden diskutierten Stoffklasse zu (c) zukommen.

### Mischungskomponenten zu (c), d.h. P, N und gewünschtenfalls weitere Makro- und/oder Mikronährstoffe für das Pflanzenwachstum enthaltende Trägerstoffe

Die Lehre der Erfindung sieht schließlich vor, in das zu behandelnde Substrat ausgewählte Wertstoffe bzw. Wertstoffgemische aus dem Bereich der Düngemittel einzutragen, die sowohl Phosphor als auch Stickstoff enthalten. Gewünschtenfalls können in diesem Zusammenhang - d.h. als anteilige Bestandteile der Komponente (c) - weitere Makro- und/oder Mikronährstoffe für das Pflanzenwachstum enthaltende Trägerstoffe zum Einsatz kommen. Zunächst einmal gilt hier allerdings:

Der Eintrag dieser Wertstoffkomponente(n) zu (c) kann gleichzeitig und verbunden mit dem Eintrag der Wertstoffe zu (b) und der dafür eingesetzten ökologisch verträglichen Netzmittel zu (a) erfolgen. Möglich ist aber auch der zeitlich versetzte Eintrag dieser Wertstoffkomponenten zu (c) oder aber auch eine Kombination eines solchen zeitlich versetzten Eintrages mit dem gleichzeitigen Eintrag der Komponenten zu (a), (b) und (c).

In einer besonders wichtigen Ausführungsform der Erfindung ist vorgesehen, als wenigstens anteilig lipophile Reste aufweisende Komponente (c) Öl-lösliche Verbindungen des P und/oder N einzusetzen. Besonders bevorzugte Vertreter dieser Hilfsstoffe sind damit die in der eingangs zitierten druckschriftlichen Veröffentlichung DE 44 37 313 beschriebenen Phospholipide und/oder deren Abkömmlinge als wesentliche Vertreter dieser Komponenten zu (c). Der Gegenstand der Offenbarung dieser DE 44 37 313 wird hiermit ebenfalls ausdrücklich zum Gegenstand der Offenbarung im Rahmen der erfindungsgemäßen Lehre gemacht, so daß nachfolgend nur noch auszugsweise wesentliche Gesichtspunkte besonders herausgestellt werden. Bereits in dieser Druckschrift wird herausgestellt, daß sich die Wirkung der zugesetzten Phospholipide auf die mikrobielle Bodenflora unter anderem darin äußert, daß im Boden vorhandene organische Verbindungen und Pflanzenreste schneller abgebaut werden, wobei es zu einer Zunahme an Bodenbakterien kommt. Im Sinne der erfindungsgemäßen Lehre sind jetzt als Kohlenstofflieferanten für das Mikroorganismenwachstum zusätzlich die lipophilen und fließfähigen Komponenten zu (b) zur Verfügung gestellt. Lipophile Molekülanteile der Komponenten gem. (c) assoziieren sich mit den lipophilen Resten vom Kohlenwasserstofftyp aus den C-Lieferanten zu (b) im Sinne der erfindungsgemäßen Lehre. In nicht vorhersehbarer Weise findet dabei eine Mobilisierung und Stärkung gerade der Mikroorganismenstämme der vielgestaltigen im Boden lebenden Populationen statt, die - im Austausch mit der Pflanzenwurzel - zur nachhaltigen Stärkung und Steigerung des Pflanzenwachstums führen. Es leuchtet ein, daß hierdurch die Wachstumsbeschleunigung wenigstens in ihren Anfangsphasen unabhängig von den im Boden vorliegenden organischen Verbindungen wie Pflanzen- bzw. Wurzelresten und dergleichen wirkt. Gleichwohl wird im weiteren Verlauf auch hier der im Boden ablaufende Kompostierungsprozeß (Mineralisierung) beschleunigt und abgestorbenes Pflanzenmaterial schneller dem biologischen Kreislauf wiederzugeführt. Im Substrat festgelegte Pflanzennährstoffe werden wieder pflanzenverfügbar. Die Durchlüftung des Bodens bzw. des Substrats, auf dem die Pflanzen wachsen, wird verbessert, der Wasserhaushalt wird gleichmäßiger gestaltet.

Bevorzugte Komponenten zu (c) sind Lecithin, Lecithin-Hydrolysate und/oder chemisch modifizierte Lecithine, die bevorzugt in Abmischung mit weiteren N-haltigen Trägerstoffen eingesetzt werden. Besonders geeignet sind hier Harnstoff und Harnstoffderivate. Als besonders leicht zugänglich für die Steuerung und Begünstigung des Mikroorganismenwachstums durch diese Komponente (c) hat sich Harnstoff als N-Lieferant erwiesen, der gleichzeitig einen Konservierungseffekt gegen nicht gewünschten mikrobiellen Befall im Nährstoffkonzentrat erzielt, so daß entsprechende Konzentrate eine hohe Lagerstabilität aufweisen. Zur Ausbildung von Harnstoffderivaten, die als N-Quelle zur Pflanzenaufzucht eingesetzt werden können, wird auf das allgemeine Fachwissen verwiesen. Lediglich beispielhaft sei hier benannt die DE 196 13 794.

Wie zuvor angegeben sind die hier im einzelnen benannten Vertreter für P und/oder N liefernde Komponenten im Sinne der Wertstoffklasse zu (c) besonders bevorzugte Vertreter. Die erfindungsgemäße Lehre ist darauf nicht eingeschränkt. Grundsätzlich können im hier betroffenen Zusammenhang auch bekannte Wasser- und/oder Öl-lösliche Verbindungen des P und/oder des N mitverwendet werden, wie sie aus der konventionellen Düngemitteltechnologie bekannt sind.

Wie schon zuvor angegeben, können die erfindungsgemäß zum Einsatz kommenden Wertstoffe bzw. Wertstoffgemische zu (c) gemeinsam mit den Komponenten zu (a) und (b), aber auch davon getrennt und hier insbesondere auch zeitversetzt zum Einsatz kommen. Ist an einen solchen getrennten Einsatz der Wertstoffe bzw. Wertstoffgemische zu (c) gedacht, dann empfiehlt sich auch hier der Einsatz entsprechender wäßriger Zubereitungen, so wie es im einzelnen in der bereits mehrfach genannten DE 44 37 313 geschildert ist. Für diesen Fall wird in der Regel auch hier die Mitverwendung tensidischer Hilfsstoffe in den wäßrigen Zubereitungen vorgesehen, wobei hier insbesondere Komponenten der erfindungsgemäß definierten Stoffklasse zu (a) in Betracht kommen, die allerdings in vergleichsweise geringen Mengen mitverwendet werden. Für solche getrennt auf das Substrat aufzubringenden wäßrigen Wertstoffgemische der Komponenten zu (c) kann dementsprechend insbesondere die Mitverwendung begrenzter Mengen an APG-Verbindungen vom O/W-Typ in Betracht kommen. Im einzelnen kann auf die in diesem Zusammenhang zuvor gemachten Sachangaben verwiesen werden.

Zu geeigneten Anwendungskonzentrationen dieser insbesondere P und N liefernden Hilfsstoffe ist - im Vergleich mit den konkreten Sachangaben des relevanten druckschriftlichen Standes der Technik - eine wichtige Abweichung gegeben: Die Lehre der DE 44 37 313 sieht vor, die entsprechenden Hilfsstoffe auf Basis von Phospholipiden in Mengen von etwa 0,5 bis 30 g pro m² Substratoberfläche aufzubringen und in das Substrat einzutragen. Als bevorzugter Mengenbereich für den Auftrag des Phospholipids sind die Grenzwerte von 1 bis 20 g/m² Substratoberfläche benannt.

Demgegenüber sieht die jetzt erfindungsgemäß modifizierte technische Lehre die Möglichkeit zur deutlichen Absenkung der Zugabe dieser Wertstoffkomponenten zur Unterklasse (c) vor. In der Regel sind hier Einsatzmengen geeignet, die deutlich unterhalb den konkreten Zahlenwerten des druckschriftlichen Standes der Technik liegen können. So sind bevorzugte Auftragskonzentrationen für diese Wertstoffe zur Unterklasse (c) wenigstens um etwa eine 10er-Potenz niedriger als im zitierten druckschriftlichen Stand der Technik angegeben. In der breitesten Fassung sieht diese bevorzugte Ausführungsform der erfindungsgemäße Lehre vor, beim Einsatz von Phospholipiden als P- und N-Lieferant diese Komponenten in Mengen von 0,01 bis 10 g/m² Substratoberfläche und vorzugsweise in Mengen von 0,1 bis 5 g/m² Substratoberfläche, einzutragen, wobei weiterhin bevorzugte Obergrenzen für die Menge der hier aufzubringenden Komponenten zu (c) bei 3 g/m² oder auch schon bei 1 g/m² Substratoberfläche liegen.

Das Mengenverhältnis der im erfindungsgemäßen Sinne bevorzugt aufzubringenden Komponenten zu (b) wird durch die im jeweiligen Einzelfall eingesetzte Menge der Komponente(n) zu (c) entscheidend mitbestimmt: Die den Kohlenstoffbedarf der Mikroorganismen abdeckenden Komponenten zu (b) werden in solchen Mengenverhältnissen zu den Komponenten (c) eingesetzt, daß - wie zuvor schon erläutert - Gewichtsverhältnisse von C:P wenigstens im Bereich von etwa 10 bis 50:1 und vorzugsweise wenigstens im Bereich von 80 bis 150:1 sichergestellt sind. Dabei kann der Einsatz wesentlich höherer Mengen der Komponenten zu (b) sinnvoll sein, so daß beispielsweise das Gewichtsverhältnis von C:P Werte im Bereich von 500:1 oder auch noch darüber erreicht. In diese Berechnung können in der tensidischen Komponente zu (a) gegebenenfalls vorliegende Kohlenwasserstoffreste mit Fettcharakter einbezogen werden, eine vergleichbar rasche Abbaubarkeit dieser tensidischen Komponenten zu (a) vorausgesetzt. Als weiterführende bevorzugte Arbeitsregel der in den erfindungsgemäßen Mehrstoffmischungen einzusetzenden Anteile von Phosphor, Stickstoff und abbaubarem Kohlenwasserstoffresten im erfindungsgemäßen Sinne gilt, daß wenigstens Gewichtsverhältnisse von P:N:C im Bereich von etwa 1:10:10 bis etwa 1:10:100 sichergestellt werden sollten.

Zeitlich getrennt und/oder gemeinsam mit den erfindungsgemäß eingetragenen Wertstoffgemischen der Komponenten zu (a) bis (c) können weitere übliche Makro- und/oder Mikronährstoffe für die Pflanzenaufzucht in die zu behandelnden Substratbereiche eingetragen werden. Auch in diesem Zusammenhang kann auf das umfangreiche allgemeine Fachwissen zur Pflanzendüngung verwiesen werden, s. hierzu beispielsweise die eingangs genannte WO 93/01150 und die umfangreiche einschlägige Fachliteratur. Als Beispiel hierzu wird verwiesen auf das Landwirtschaftliche Lehrbuch K.-U. Heyland "Allgemeiner Pflanzenbau", Verlag Eugen Ulmer, Stuttgart, 7. Auflage, Seite 255. Makronährstoffe sind bekanntlich neben den bereits genannten Elementen insbesondere Kalium, Magnesium, Kalk und Schwefel. Zu den Mikronährstoffen oder auch Spurenelementen gehören Bor, Kupfer, Eisen, Mangan und zahlreiche weitere der Fachwelt bekannte Komponenten.

Eine wichtige Ausführungsform der erfindungsgemäßen Lehre sieht die Mitverwendung ausgewählter und das gesunde Pflanzenwachstum fördernder Mikroorganismenstarterkulturen vor, die insbesondere aus den Bereichen entsprechender Bakterien- und/oder Mykorrhiza-Stämme ausgewählt sein können. Durch den Einsatz solcher ausgewählter Starterkulturen und der erfindungsgemäßen Stärkungsmittel zur raschen Entwicklung solcher ausgewählten Mikroorganismenstämme kann eine Optimierung ausgewählter Mikroorganismenpopulationen im Substrat erzielt und damit die angestrebte sekundäre Förderung des Pflanzenwachstums optimiert werden.

Die Anwendung des erfindungsgemäß definierten Wertstoffgemisches und das damit zum Einsatz kommende Wirkungsprinzip der gezielten Förderung des Wachstums der erwünschten Mikroorganismenflora im Boden, führt zu Einsatzgebieten der Praxis in breitester Streuung. Zuvor sei in diesem Sachzusammenhang aber noch auf den folgenden wichtigen Gesichtspunkt verwiesen: Wertstoffgemische im Sinne der Erfindung können ohne Mitverwendung von Düngemitteln bzw. Düngemittelkomponenten tierischen Ursprungs aufgebaut sein. Die erfindungsgemäßen Wertstoffgemische können rein pflanzlichen Ursprungs und gegebenenfalls pflanzlich-mineralischen Ursprungs sein. Irgendwelche potentiellen seuchenhygienischen Bedenken sind damit von vornherein ausgeschlossen.

Für die Anwendungsbereiche erschließt sich ein breites Feld, wobei jeweils das Prinzip der Förderung der gesunden mikrobiologischen Wachstumsprozesse in dem Substrat bzw. der Bodenstruktur im Sinne einer Bodenkur im Vordergrund stehen. Ohne Anspruch auf Vollständigkeit sind hier im einzelnen die folgenden Einsatzgebiete zu nennen:

Erdbodenkur mit rottebeschleunigender Wirkung (Mineralisierung), Bodenkur mit bodenverbessernder Wirkung (Verbesserung der Bodenbelüftung, Beseitigung von anaeroben Bodenbereichen), Bodenkur mit pflanzenstärkender Wirkung, wobei hier insbesondere eine durch natürliche Mikroorganismenwachstumsprozesse ausgelöste slow-release-Düngewirkung im Vordergrund stehen kann.

Einem weiteren Gesichtspunkt kommt besondere Bedeutung zu: Die Stärkung und Gesundung des erwünschten Mikroorganismenwachstums im Substrat führt zur Stärkung der Abwehrkraft der in diesem Substrat wachsenden Pflanzen gegen Krankheits- und Schädlingsbefall. In ihrer Abwehrkraft gegen Schädlingsbefall werden dabei sowohl der Wurzelbereich als auch der Pflanzenbereich oberhalb der Bodenfläche gestärkt. Der hier insgesamt ausgelösten Boden- und Pflanzenstärkung kommt nachhaltige Wirkung zu. Das erfindungsgemäß gesteuerte und geförderte Mikroorganismenwachstum führt ja nicht nur in der Lebensphase der Mikroorganismen zu den gewünschten Anregungen der gesamten Mikroflora und Bodenfauna, auch die im Boden zurückbleibende Biomasse des jeweils absterbenden Anteils der Mikroorganismen steht als wertvolles Düngemittelgemisch für weiterführende Wachstumsprozesse im Mikroorganismen- und Pflanzenbereich zur Verfügung. So erschließen sich durch die erfindungsgemäße Lehre neue Möglichkeiten der Gesunderhaltung und Gesundung von landwirtschaftlich und/oder forstwirtschaftlich genutzten Erdreichsubstraten.

### Beispiel

Die im nachfolgenden geschilderten Untersuchungen zur Verbesserung des Pflanzenwachstums durch Applikation der erfindungsgemäßen Wertstoffe bzw. Wertstoffgemische zu (a), (b) und (c) sind auf einer bisher in konventioneller Weise gepflegten Golfanlage durchgeführt worden. Die Applikation der Wertstoffgemische erfolgt dabei auf den sogenannten Golf-Grüns. Ausgewählt wurden dabei insbesondere solche Rasenbereiche, die trotz regelmäßiger konventioneller Pflege Schadstoffstellen aufwiesen. Zu nennen sind in diesem Zusammenhang insbesondere die sogenannten Trockenstellen (Dry-Patches), Grün-Bereiche mit ausgeprägter Ausbildung des sogenannten Rasenfilzes sowie schließlich Schadstoffstellen, die in bekannter Weise durch starken Befall von Schadpilzen (Pythium, Ophiobulus usw.) befallen sind. Es ist bekannt, daß durch konventionelle Bewässerung einschl. des Auftrages von Fungiziden die hier genannten Schäden bzw. Schadstoffstellen in der Regel nicht oder nur begrenzt beseitigt werden können. Schadorganismen im Boden und den Rasentragsschichten führen hier primär zu einer partiellen Hydrophobierung des Bodens, wäßrige Nährstofflösungen können nicht wieder in das Grün eindringen. Rasenfilz bildet sich sehr schnell auf den Grüns und anderen Rasenflächen, weil sich die Wurzelmasse des Rasens schnell umbaut und auch teilweise Schnittgut liegen bleibt. Die biologische Aktivität der Bodenbakterien wird eingeschränkt. Damit stellt der Rasenfilz eine nicht unerhebliche Quelle für Pilzinfektionen der Gräser dar. Erschwerend fällt hier häufig ins Gewicht, daß die Rasentragsschichten vergleichsweise nährstoffarm aufgebaut sind und folglich die biologische Aktivität der Bakterienstämme gering ist.

Im Sinne der erfindungsgemäßen Lehre werden wäßrige Zubereitungen der nachfolgenden Wertstoffkonzentrate in die der Untersuchung zugrundegelegten Rasenflächen eingetragen:

### (1) Wertstoffkonzentrat zu den erfindungsgemäß definierten Komponenten zu (a) und (b):

45 Gew.-% APG als Netzmittel vom O/W-Typ mit einem APG-Aktivsubstanzgehalt von 60 Gew.-%, zum Rest Wasser (Handelsprodukt APG 220 UP der Anmelderin mit überwiegend C_{8/10}-Fettalkoholresten in der APG-Struktur)
5 Gew.-% C_{16/18}-Fettalkohol (Handelsprodukt HD Ocenol 80/85 der Anmelderin, Jodzahl 80 bis 85)
15 Gew.-% Glucerinmonooleat (Handelsprodukt Edenor GMO der Anmelderin)
15 Gew.-% Ethanol
zum Rest Wasser.

### (2) Wertstoffkonzentrat zu der erfindungsgemäß definierten Komponente zu (c):

19,9 Gew.-% Harnstoff (N-Quelle)
19,8 Gew.-% enzymatisch hydrolysiertes Sojalecithin (Handelsprodukt Lipotin NE) als N- und P-Quelle
1,0 Gew.-% des unter dem Handelsnamen "APG 600" vertriebenen O/W-Netzmittels auf APG-Basis mit überwiegend C_{12/14}-Fettalkoholresten als Emulsionsstabilisator
zum Rest Wasser.

In einem ersten Arbeitsschritt wird das Wertstoffgemisch zu (1) - die Komponenten zu (a) und (b) - in wäßriger Verdünnung mittels eines Hydro-Ject in die zu behandelnden Grünflächen eingetragen. Dabei wird die wäßrig verdünnte Wertstoffmischung in Form feiner Wasserstrahlen in den Boden geschossen. Die Wasserstrahlen werden im Boden gebrochen, die wäßrige Lösung verteilt sich darin, gleichzeitig wird eine Bodenbelüftung ausgelöst. Das Wertstoffgemisch zu (1) wird dabei in einer Menge von 2 cm³ Konzentrat der zuvor angegebenen Zusammensetzung/m² Bodenfläche ausgebracht.

Innerhalb weniger Tage ist bereits eine wesentliche Erholung der behandelten Grünstellen sichtbar. Keine Wirkung zeigt sich allerdings an den von Pilzkrankheiten befallenen Schadstellen. Selbst der Einsatz von Fungiziden zeigt hier kaum Wirkung.

Nach Ablauf von 14 Tagen wird eine wäßrige Lösung des Wertstoffgemisches zu (2) in einer Einsatzmenge von 20 g/m² - Mengenangabe auch hier bezogen auf das Wertstoffkonzentrat - ausgebracht. Dabei wird die zuvor mit dem Wertstoffgemisch zu (1) behandelte Rasenfläche streifenförmig mit dem Wertstoffgemisch zu (2) derart belegt, daß sich jeweils streifenförmige Bodenbereiche aneinander anschließen die einmal mit beiden Wertstoffkonzentraten behandelt worden sind und zum anderen nur mit dem Wertstoffkonzentrat zu (1) behandelt worden sind. Dabei wird darauf geachtet, daß diese streifenförmige Belegung auch gerade die Trockenstellen einschl. der von Schadpilzen befallenen Stellen überdeckt.

Schon nach 10 Tagen kann eine deutliche Verbesserung des Rasenwachstums in den Bodenbereichen festgestellt werden, die sowohl mit der Wertstoffkombination zu (1) als auch mit der Wertstoffkombination zu (2) behandelt worden sind. Die farbliche Ausbildung dieser Grünbereiche ist nicht nur deutlich grüner als die der anderen Grünbereiche, die Grasnarbe wird deutlich dichter, die Schnittgutmenge erhöht sich. Zum Nachweis dieses Sachverhalts wurden die betroffenen Grünbereiche per Hand gemäht und keinerlei weiteren Nährstoffe auf die betroffenen Grünbereiche aufgebracht.

Besonders eindrucksvoll zeigt sich die synergistische Wirkungssteigerung an den sogenannten Rasenringen, die von den angesprochenen Rasenkrankheiten ausgebildet worden waren. Auch diese Bereiche ergrünten in den doppelt belegten Streifen mit der Gesamtkombination der Wertstoffe zu (a), (b) und (c), obwohl vorher hier keine Gräser mehr standen.

Über diese visuell wahrzunehmenden Effekte hinausgehend zeigte sich folgendes: Die erfindungsgemäß behandelten Grünanteile wiesen unter den klimatischen Bedingungen der Versuchsperiode am Morgen keinen Tau-Befall auf, obwohl es zu diesem Zeitpunkt hier gerade sehr stark taute - festzustellen auf den nicht behandelten Grünbereichen. Hier ist offensichtlich ein Hinweis auf das stark aktivierte Wachstum der Mikroorganismenflora gegeben. Das Wachstum dieser Mikroorganismenflora setzt bekanntlich neben der Bildung von CO₂ und H₂O Energie frei. Der unter den Arbeitsbedingungen freigewordene Energiebetrag dürfte für diese Freiheit von Tau-Befall verantwortlich sein.

### Beispiel 2

Auf einem landwirtschaftlichen Versuchsgut werden Freilandversuche zum Maisanbau durchgeführt, wobei in einer Mehrzahl von Varianten gem. der nachfolgenden Tabelle 1 Wertstoffe bzw. Wertstoffgemische im Sinne der zuvor definierten Komponenten zu (a), (b) und (c) zum Einsatz kommen und dabei mit nicht behandelten Parzellen verglichen werden. Im einzelnen gilt hier:

Die Parzellengröße beträgt jeweils 6 x 12 m pro Parzelle, jede Variante in 4-facher Wiederholung bei randomisierter Parzellenanordnung.

Neben den Kontroll-Parzellen ohne Einsatz der erfindungsgemäß beschriebenen Wertstoffe bzw. Wertstoffgemische werden - unter Bezugnahme auf die Angaben des vorherigen Beispiels 1 die nachfolgenden Komponenten bzw. Komponentengemische ausgebracht:

### (1) Wertstoffkonzentrat zu den erfindungsgemäß definierten Komponenten zu (a) und (b):

Zusammensetzung gem. den entsprechenden Angaben des Beispiels 1.

### (2) Wertstoffkonzentrat zu der erfindungsgemäß definierten Komponente zu (c):

Das in Beispiel 1 genannte Wertstoffgemisch wird durch Mitverwendung von Kaliumpyrophosphat leicht variiert. Im einzelnen gelten die folgenden Angaben:
20 Gew.-% Harnstoff (N-Quelle)
20 Gew.-% des Handelsprodukts "Lipotin NE"
7 Gew.-% K₄P₂O₇
2 Gew.-% des O/W-Netzmittels auf APG-Basis (Handelsprodukt "APG 600")
   zum Rest Wasser.

Diese Wertstoffkonzentrate zu (1) und (2) kommen dabei sowohl jeweils alleine als auch in Abmischung miteinander zum Einsatz, wobei jeder dieser Versuchsansätze in unterschiedlichen Konzentrationen der aufgetragenen Wertstoffe bzw. Wertstoffgemische erfolgt. Die jeweiligen Versuchsansätze sind dabei in der nachfolgenden Tabelle 1 zusammengefaßt. Die Ausbringung des Saatgutes auf sämtlichen Versuchsparzellen erfolgte am gleichen Tag. Der Auftrag der Wertstoffe bzw. Wertstoffgemische wurde am nachfolgenden Tag vorgenommen.

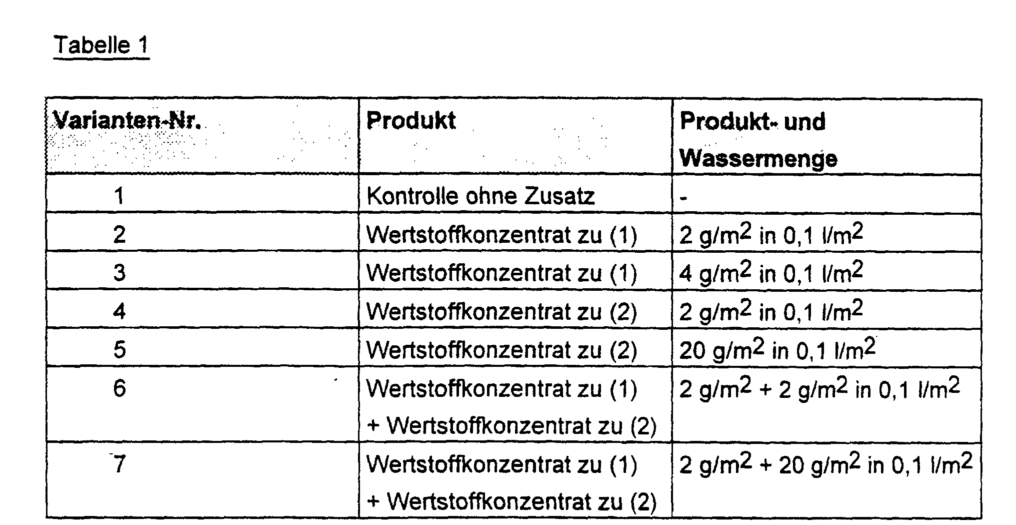

Auf den zum Vergleich vorliegenden Parzellen dieses Versuchsansatzes werden zunächst Messungen der Infiltrationsrate des Bodens vor Ort (im Feld) mehrfach während der Vegetationsperiode zur Erfassung der pro Zeiteinheit versickernden Wassermenge als Maß für den Luft- und Wasserhaushalt des Bodens vorgenommen. Die Wertebestimmung wird dabei jeweils mit einem "Doppelring-Infiltrometer" (Hersteller Fa. Eijkelkamp, Agrisearch Equipment, Giesbeek, Niederlande) vorgenommen.

Eine erste Wertebestimmung findet dabei 12 Tage nach der Produktapplikation statt. Die nachfolgende Tabelle 2 faßt die Mittelwerte der dabei jeweils bestimmten Maßzahlen zusammen.

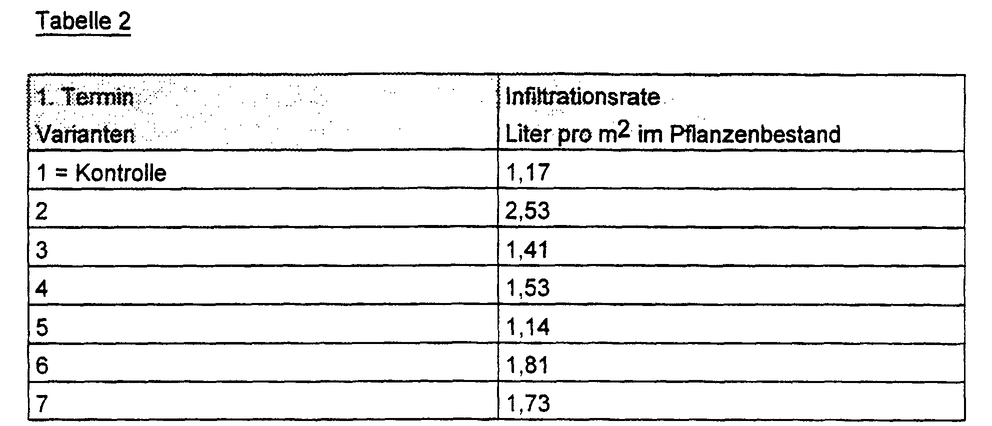

Eine zweite Bestimmung der Infiltrationsrate wird 3 Monate nach der Produktapplikation wiederum vor Ort (im Feld) vorgenommen. Die hierbei erhaltenen Ergebnisse sind in der nachfolgenden Tabelle 3 zusammengefaßt.

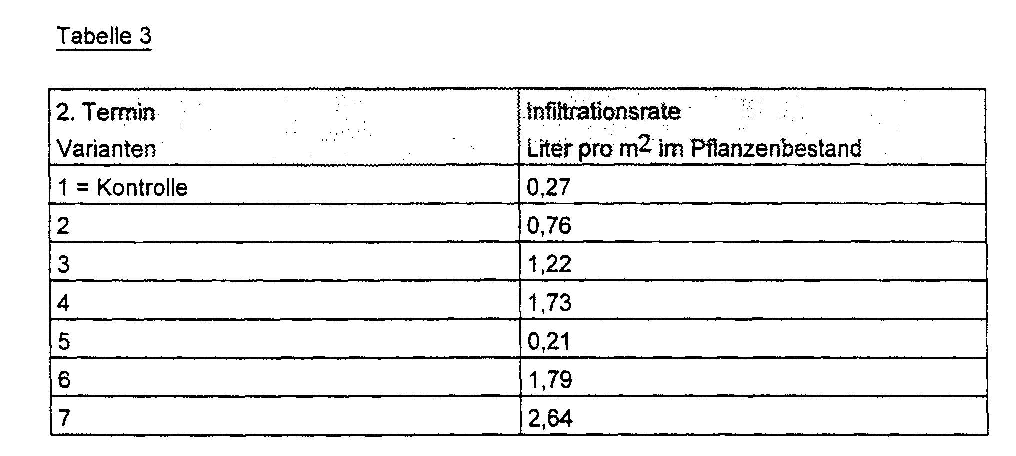

Der Vergleich der Zahlenwerte aus den Tabellen 2 und 3 zeigt, daß durch das getrennte Aufbringen der tensidischen Wertstoffkomponente zu (1) kurzfristig durch die tensidische Wirkung eine Verdoppelung der Infiltrationsrate erreicht werden kann - Tabelle 2, Variante 2 - die jedoch nicht nachhaltig ausgebildet ist. Die nachhaltige deutliche Verbesserung der Infiltrationsrate wird durch die erfindungsgemäß definierten Wertstoffgemische zu den Einzelkomponenten zu (a), (b) und (c) erreicht, s. hierzu die Angaben der Tabelle 3 zu den Varianten 6 und 7. Hier zeigen sich die Auswirkungen des erfindungsgemäßen Arbeitsprinzips der gesunden Wachstumssteigerung der Erdboden-Mikroorganismenflora und der damit verbundenen Anregung und Entwicklung der Erdbodenfauna.

Auch weitere Messungen der Aggregatstabilität an gezogenen Bodenproben - mehrfach während der Vegetationsperiode - als Maß für die Neigung zur Oberflächenverschlämmung eines Bodens zeigen deutlich erhöhte Percolatwerte für die erfindungsgemäß behandelten Bodenproben in 0 bis 2 cm Tiefe sowie 2 bis 6 cm Tiefe im Vergleich zu entsprechenden Bodenproben aus der Kontrollvariante bzw. den Varianten zu 2 bis 5 aus der zuvor gebrachten Tabelle 1.

## Patentansprüche

1. Verfahren zur Wachstumssteigerung der Erdreich-Mikroorganismenflora und Förderung des mikrobiellen Aufschlusses eines mit organischer Substanz beladenen Erdreichs (Substrat) sowie damit verbundener nachhaltiger Stärkung der Pflanzengesundheit und Förderung eines gesunden Pflanzenwachstums in diesem Substrat,
**gekennzeichnet durch**
den Eintrag wäßriger Zubereitungen, enthaltend
(a) ökologisch verträgliche Netzmittel vom O/W-Typ zusammen mit
(b) lipophile gesättigte und/oder olefinisch ungesättigte Kohlenwasserstoffreste mit Fettstruktur aufweisenden und sowohl aerob als auch anaerob abbaubaren organischen Verbindungen als zusätzliche C-Lieferanten für das Wachstum der Mikroorganismenflora,
verbunden mit gleichzeitigem und/oder zeitlich versetztem Eintrag von
(c) wenigstens anteilig lipophile Reste aufweisende Verbindungen des P und/oder N und gewünschtenfalls weitere Makro- und/oder Mikronährstoffe für das Pflanzenwachstum enthaltenden Trägerstoffen.

2. Verfahren nach Anspruch 1, **dadurch gekennzeichnet, daß** als Komponente (a) biologisch abbaubare Tenside wenigstens überwiegend nichtionischen Charakters eingesetzt werden, die weiterhin bevorzugt zum wenigstens überwiegenden Anteil Naturstoff-basierten Ursprungs sind und dabei bevorzugte HLB-Werte im Bereich von 10 bis 18 aufweisen.

3. Verfahren nach Ansprüchen 1 und 2, **dadurch gekennzeichnet, daß** als Komponente (a) wenigstens anteilsweise, bevorzugt wenigstens überwiegend Alkyl(oligo)glukosid-Verbindungen (APG-Verbindungen) eingesetzt werden, deren Alkylrest sich wenigstens überwiegend von geradkettigen Fettalkoholen ableitet.

4. Verfahren nach Ansprüchen 1 bis 3, **dadurch gekennzeichnet, daß** APG-Verbindungen aus Glukose und insbesondere Naturstoff-basierten Fettalkoholen mit wenigstens 6 C-Atomen, vorzugsweise mit 8 bis 24 C-Atomen und DP-Werten im Bereich von 1,2 bis 5 eingesetzt werden.

5. Verfahren nach Ansprüchen 1 bis 4, **dadurch gekennzeichnet, daß** als Komponente (b) Öl-lösliche, dabei jedoch biologisch verträgliche organische Verbindungen mit aliphatischen und/oder olefinisch ungesättigten, bevorzugt wenigstens überwiegend geradkettigen Kohlenwasserstoffresten mit wenigstens 6 C-Atomen und insbesondere mit wenigstens 8 C-Atomen eingesetzt werden.

6. Verfahren nach Ansprüchen 1 bis 5, **dadurch gekennzeichnet, daß** Komponenten zu (b) eingesetzt werden, die wenigstens anteilsweise mit Sauerstoff als Heteroatom funktionalisiert sind, wobei der Einsatz von Fettalkoholen und/oder Fettsäuren bzw. ihren Derivaten, wie ihren Estern bzw. Partialestern, Ethern und/oder Amiden, bevorzugt ist.

7. Verfahren nach Ansprüchen 1 bis 6, **dadurch gekennzeichnet, daß** die Komponenten zu (b) wenigstens überwiegend Naturstoff-basiert sind.

8. Verfahren nach Ansprüchen 1 bis 7, **dadurch gekennzeichnet, daß** die Komponenten zu (b) wenigstens anteilsweise Stockpunkte gleich/kleiner 25 bis 30°C und insbesondere gleich/kleiner 10 bis 15°C aufweisen.

9. Verfahren nach Ansprüchen 1 bis 8, **dadurch gekennzeichnet, daß** als Komponenten zu (b) olefinisch ungesättigte C₁₂₋₂₄-Fettalkohole natürlichen Ursprungs, insbesondere wenigstens überwiegend C_{16/18}-Fettalkohole mit hohem Grad olefinischer Doppelbindungen und Erstarrungsbereichen gleich/kleiner 20°C, vorzugsweise gleich/kleiner 10 bis 15°C, und/oder Fettsäurepartialester wie Glycerinmonooleat eingesetzt werden, wobei Abmischungen solcher Komponenten zu (b) bevorzugt sein können.

10. Verfahren nach Ansprüchen 1 bis 9, **dadurch gekennzeichnet, daß** die Fettalkohole und Partialester in Mischungsverhältnissen von 1:1 bis 1:10, vorzugsweise von 1:1 bis 1:5 und insbesondere von 1:1 bis 1:3 Gewichtsteilen eingesetzt werden.

11. Verfahren nach Ansprüchen 1 bis 10, **dadurch gekennzeichnet, daß** als wenigstens anteilig lipophile Reste aufweisende Verbindungen der Komponente (c) Öl-lösliche Verbindungen des P und/oder N eingesetzt werden.

12. Verfahren nach Ansprüchen 1 bis 11, **dadurch gekennzeichnet, daß** als wenigstens anteilig lipophile Reste aufweisende Verbindungen der Komponente (c) Öl-lösliche Verbindungen des P und/oder N eingesetzt werden.

13. Verfahren nach Ansprüchen 1 bis 12, **dadurch gekennzeichnet, daß** als Komponente (c) Lecithin, Lecithin-Hydrolysate und/oder chemisch modifizierte Lecithine - bevorzugt in Abmischung mit weiteren N-haltigen Makronährstoffen - eingesetzt werden, wobei insbesondere Harnstoff und/oder Harnstoffderivate als weitere N-haltige Komponenten verwendet werden können.

14. Verfahren nach Ansprüchen 1 bis 13, **dadurch gekennzeichnet, daß** auch die Komponenten zu (c) in der Form wäßriger Zubereitungen und dabei vorzugsweise auch hier unter Mitverwendung ökologisch verträglicher Netzmittel in das Erdreichsubstrat eingetragen werden.

15. Verfahren nach Ansprüchen 1 bis 14, **dadurch gekennzeichnet, daß** die Komponenten zu (b) und - bei biologischer Abbaubarkeit der Komponenten zu (a) - die Abmischungen der Komponenten zu (b) und (a) in solchen Mengenverhältnissen zu den Komponenten (c) eingesetzt werden, daß Gewichtsverhältnisse von C:P wenigstens im Bereich von 10 bis 50:1, vorzugsweise wenigstens im Bereich von 80 bis 150:1 sichergestellt sind, dabei aber auch Werte bis zu 500:1 und darüber erreichen können.

16. Verfahren nach Ansprüchen 1 bis 15, **dadurch gekennzeichnet, daß** im praktischen Einsatz über die Mengenabstimmung der in das Substrat eingetragenen Komponenten (a)/(b) und (c) Gewichtsverhältnisse von P:N:C im Bereich von wenigstens etwa 1:10:10 bis 1:10:100 eingestellt werden.

17. Verfahren nach Ansprüchen 1 bis 16, **dadurch gekennzeichnet, daß** beim Einsatz von Phospholipiden als P-Lieferant der Komponente zu (c) diese in Mengen von 0,01 bis 10 g/m² Substratoberfläche, vorzugsweise in Mengen von 0,1 bis 5 g/m² Substratoberfläche, und die Komponenten (a)/(b) in einer dem erwünschten Molverhältnis C:P angepaßten Menge ausgebracht werden.

18. Wasserverdünnbare wäßrige Konzentrate von Trägerstoffen für die Wachstumssteigerung der das Pflanzenwachstum fördernden Erdreich-Mikroorganismenflora, enthaltend
- wenigstens anteilig Öl-lösliche Verbindungen des P und/oder N und gewünschtenfalls weitere Makro- und/oder Mikronährstoffe für das Pflanzenwachstum aufweisende Trägerstoffe
- zusammen mit biologisch verträglichen und insbesondere mikrobiologisch abbaubaren Netzmitteln vom O/W-Typ
**dadurch gekennzeichnet,**
**daß** sie zusätzlich
- feinstteilig emulgierbare und/oder dispergierbare C-Lieferanten für das Wachstum der Mikroorganismenflora aus der Klasse sowohl aerob als auch anaerob abbaubarer und dabei lipophile gesättigte und/oder olefinisch ungesättigte Kohlenwasserstoffreste mit Fettstruktur aufweisender organischer Verbindungen enthalten.

19. Mehrstoffgemische nach Anspruch 18, **dadurch gekennzeichnet, daß** sie als C-Lieferanten mit Sauerstoff funktionalisierte und wenigstens überwiegend Fettstruktur aufweisende organische Verbindungen enthalten, die bevorzugt wenigstens einer der nachfolgenden Stoffklassen zugeordnet sind: Fettalkohole und/oder Fettsäuren bzw. deren Salze sowie deren Derivate, wie Ester bzw. Partialester mit 1- und/oder mehrwertigen Alkoholen, Ether und/oder Amide.

20. Mehrstoffgemische nach Ansprüchen 18 und 19, **dadurch gekennzeichnet, daß** sie die C-Lieferanten - in Abstimmung mit den P-haltigen Komponenten - in solchen Mengen enthalten, daß im biologischen Abbau C:P-Verhältnisse - Gewichtsmengen und bezüglich des C-Lieferanten bezogen auf die organischen Anteile mit Fettstruktur - von wenigstens 10 bis 50:1, vorzugsweise von wenigstens 80 bis 100:1 gewährleistet sind.

21. Mehrstoffgemische nach Ansprüchen 18 bis 20, **dadurch gekennzeichnet, daß** sie als Netzmittel vom O/W-Typ APG-Verbindungen aus Glukose und insbesondere Naturstoff-basierten Fettalkoholen mit wenigstens 6 C-Atomen, vorzugsweise 8 bis 24 C-Atomen, bei DP-Werten im Bereich von 1 bis 5, insbesondere im Bereich von 1,2 bis 3, und HLB-Werten im Bereich von 10 bis 18 enthalten.

22. Mehrstoffgemische nach Ansprüchen 18 bis 21, **dadurch gekennzeichnet, daß** sie P und N aufweisende Mischungskomponenten enthalten, die wenigstens anteilsweise in Form lipophiler organischer Verbindungen ausgebildet sind, wobei der Einsatz entsprechender Phospholipide bevorzugt sein kann.

23. Mehrstoffgemische nach Ansprüchen 18 bis 22, **dadurch gekennzeichnet, daß** sie als Phospholipide Lecithin, Lecithinhydrolysate und/oder chemisch modifizierte Lecithine enthalten.

24. Mehrstoffgemische nach Ansprüchen 18 bis 23, **dadurch gekennzeichnet, daß** sie zusätzlich Harnstoff und/oder Harnstoffderivate als N-Quelle enthalten.

25. Anwendung des Arbeitsprinzips einer gesunden Wachstumssteigerung der Erdboden-Mikroorganismenflora und der damit verbundenen Anregung und Entwicklung der Erdbodenfauna durch Eintrag insbesondere wäßriger Zubereitungen von wenigstens anteilsweise Öl-löslichen Verbindungen des P und/oder N und gewünschtenfalls weitere Makro- und/oder Mikronährstoffe für das Pflanzenwachstum enthaltenden Trägerstoffen, gleichzeitig mit und/oder zeitversetzt zum zusätzlichen Eintrag von C-Lieferanten für das Wachstum der Mikroorganismenflora auf Basis sowohl aerob als auch anaerob abbaubarer organischer Verbindungen, enthaltend lipophile gesättigte und/oder olefinisch ungesättigte Kohlenwasserstoffreste mit Fettstruktur, für eine Erdbodenkur mit rottebeschleunigender Wirkung (Mineralisierung), mit bodenverbessernder Wirkung (Verbesserung der Bodenbelüftung, Beseitigung von anaeroben Bodenbereichen) und insbesondere als Vitalisierungsmittel mit pflanzenstärkender bevorzugt slow-release-Düngewirkung.

26. Anwendung nach Anspruch 25, **dadurch gekennzeichnet, daß** wenigstens die C-Lieferanten für das Wachstum der Mikroflora zusammen mit mikrobiologisch abbaubaren Netzmitteln vom O/W-Typ vorliegen und als verdünnte wäßrige Lösung, Emulsion und/oder Suspension in das Erdreich eingetragen werden.

27. Anwendung nach Ansprüchen 25 und 26 zur Zulieferung und Anreicherung von insbesondere aerob abbaubaren C-Lieferanten in den Rhizospheren-Bereich und damit in den unmittelbaren Kontakt mit der Pflanzenwurzel-Oberfläche.

28. Anwendung nach Ansprüchen 25 bis 27, **gekennzeichnet durch** die Mitverwendung von das gesunde Pflanzenwachstum fördernden Mikroorganismen-Starterkulturen, insbesondere aus den Bereichen entsprechender Bakterien- und/oder Mykorrhiza-Stämme.

## Claims

1. A process for increasing the growth of soil microorganism flora and promoting the microbial break-up of soil (substrate) polluted with an organic substance and - in association therewith - durably improving plant health and promoting healthy plant growth in that substrate, **characterized by** the introduction of aqueous preparations containing
(a) ecologically safe wetting agents of the o/w type together with
(b) aerobically and anaerobically degradable organic compounds containing lipophilic saturated and/or olefinically unsaturated hydrocarbon radicals of fatty structure as additional C sources for the growth of the microorganism flora,
in conjunction with the simultaneous and/or staggered introduction of
(c) supporting materials containing compounds of P and/or N at least partly bearing lipophilic radicals and, if desired, other macro and/or micro nutrients for plant growth.

2. A process as claimed in claim 1, **characterized in that** at least predominantly nonionic biodegradable surfactants which are preferably at least predominantly based on natural substances and which have preferred HLB values in the range from 10 to 18 are used as component (a).

3. A process as claimed in claims 1 and 2, **characterized in that** alkyl (oligo)glucoside compounds (APG compounds) of which the alkyl group derives at least predominantly from linear fatty alcohols are used at least partly and preferably at least predominantly as component (a).

4. A process as claimed in claims 1 to 3, **characterized in that** APG compounds of glucose and, more particularly, fatty alcohols based on natural substances and containing at least 6 and preferably 8 to 24 carbon atoms and having DP values of 1.2 to 5 are used.

5. A process as claimed in claims 1 to 4, **characterized in that** oil-soluble but biologically compatible organic compounds containing aliphatic and/or olefinically unsaturated, preferably at least predominantly linear hydrocarbon radicals containing at least 6 carbon atoms and, in particular, at least 8 carbon atoms are used as component (b).

6. A process as claimed in claims 1 to 5, **characterized in that** components (b) at least partly functionalized with oxygen as hetero atom are used, the use of fatty alcohols and/or fatty acids or derivatives thereof, such as esters or partial esters, ethers and/or amides, being preferred.

7. A process as claimed in claims 1 to 6, **characterized in that** the components (b) are at least predominantly based on natural substances.

8. A process as claimed in claims 1 to 7, **characterized in that** the components (b) at least partly have pour points of or below 25 to 30°C and, more particularly, of or below 10 to 15°C.

9. A process as claimed in claims 1 to 8, **characterized in that** olefinically unsaturated C₁₂₋₂₄ fatty alcohols of natural origin, more particularly at least predominantly C_{16/18} fatty alcohols with a high degree of olefinic double bonds and solidification ranges of or below 20°C and preferably of or below 10 to 15°C, and/or fatty acid partial esters, such as glycerol monooleate, are used as component (b), mixtures of such components (b) being preferable.

10. A process as claimed in claims 1 to 9, **characterized in that** the fatty alcohols and partial esters are used in mixing ratios of 1:1 to 1:10, preferably 1:1 to 1:5 and more preferably 1:1 to 1:3 parts by weight.

11. A process as claimed in claims 1 to 10, **characterized in that** oil-soluble compounds of P and/or N are used as the compounds at least partly bearing lipophilic radicals of component (c).

12. A process as claimed in claims 1 to 11, **characterized in that** oil-soluble compounds of P and/or N are used as the compounds at least partly bearing lipophilic radicals of component (c).

13. A process as claimed in claims 1 to 12, **characterized in that** lecithin, lecithin hydrolyzates and/or chemically modified lecithins, preferably in admixture with other N-containing macro nutrients, are used as component (c), urea and/or urea derivatives in particular optionally being used as other N-containing components.

14. A process as claimed in claims 1 to 13, **characterized in that** the components (c) are also introduced into the substrate in the form of aqueous preparations, again preferably using ecologically compatible wetting agents.

15. A process as claimed in claims 1 to 14, **characterized in that** the components (b) and - assuming the components (a) are biodegradable - the mixtures of components (b) and (a) are used in such quantity ratios to the components (c) that ratios by weight of C to P of at least 10 to 50:1 and preferably of at least 80 to 150:1 are established although values of up to 500:1 or higher can also be achieved.

16. A process as claimed in claims 1 to 15, **characterized in that**, in practice, ratios by weight of P to N to C of at least about 1:10:10 to 1:10:100 are adjusted by co-ordinating the quantities of components (a)/(b) and (c) introduced into the substrate.

17. A process as claimed in claims 1 to 16, **characterized in that**, where phospholipids are used as the P source of component (c), they are applied in quantities of 0.01 to 10 g/m² substrate surface and preferably in quantities of 0.1 to 5 g/m² substrate surface while components (a)/(b) are applied in a quantity adapted to the desired molar ratio of C to P.

18. Water-dilutable aqueous concentrates of supporting materials for increasing the growth of plant-growth-promoting soil microorganism flora containing
- supporting materials at least partly containing oil-soluble compounds of P and/or N and, if desired, other macro and/or micro nutrients for plant growth
- together with biologically compatible and, in particular, microbiologically degradable wetting agents of the o/w type,
**characterized in that** they additionally contain
- fine-particle emulsifiable and/or dispersible C sources for the growth of microorganism flora from the class of aerobically and anaerobically degradable organic compounds containing lipophilic saturated and/or olefinically unsaturated hydrocarbon radicals of fatty structure.

19. Multicomponent mixtures as claimed in claim 18, **characterized in that** they contain as C sources oxygen-functionalized organic compounds at least predominantly of fatty structure which preferably belong to at least one of the following classes: fatty alcohols and/or fatty acids or salts and derivatives thereof, such as esters or partial esters with mono- and/or polyfunctional alcohols, ethers and/or amides.

20. Multicomponent mixtures as claimed in claims 18 and 19, **characterized in that** they contain the C sources - in co-ordination with the P-containing components - in such quantities that C:P ratios (quantities by weight and, in the case of the C source, based on the organic components of fatty structure) of at least 10 to 50:1 and preferably of at least 80 to 100:1 are guaranteed.

21. Multicomponent mixtures as claimed in claims 18 to 20, **characterized in that** they contain APG compounds of glucose and in particular fatty alcohols based on natural substances which contain at least 6 and preferably 8 to 24 carbon atoms as wetting agents of the o/w type, the APG compounds having DP values of 1 to 5 and preferably of 1.2 to 3 and HLB values of 10 to 18.

22. Multicomponent mixtures as claimed in claims 18 to 21, **characterized in that** they contain P- and N-containing mixture components at least partly in the form of lipophilic organic compounds, the use of corresponding phospholipids being preferable.

23. Multicomponent mixtures as claimed in claims 18 to 22, **characterized in that** they contain lecithin, lecithin hydrolyzates and/or chemically modified lecithins as phospholipids.

24. Multicomponent mixtures as claimed in claims 18 to 23, **characterized in that** they additionally contain urea and/or urea derivatives as N source.

25. The application of the working principle of healthy growth promotion of soil microorganism flora and the resulting stimulation and development of soil fauna by introduction of in particular aqueous preparations of supporting materials at least partly containing oil-soluble compounds of P and/or N and, if desired, other macro and/or micro nutrients for plant growth at the same time as and/or at a different time from the additional introduction of C sources for the growth of microorganism flora based on aerobically and anaerobically degradable organic compounds containing lipophilic saturated and/or olefinically unsaturated hydrocarbon radicals of fatty structure for soil rehabilitation with a rotting-accelerating effect (mineralization) and a soil-improving effect (improvement of soil aeration, elimination of anaerobic soil regions) and in particular as a vitalizing medium with a plant-strengthening fertilizing effect, preferably of the slow-release type.

26. The application claimed in claim 25, **characterized in that** at least the C sources for the growth of the microflora are present together with microbiologically degradable wetting agents of the o/w type and are introduced into the soil in the form of a dilute aqueous solution, emulsion and/or suspension.

27. The application claimed in claims 25 and 26 for supplying and enriching degradable C sources, particularly aerobically degradable C sources, to and in the rhizosphere region so that they come into direct contact with the surface of the plant roots.

28. The application claimed in claims 25 to 27, **characterized by** the use of microorganism starter cultures which promote healthy plant growth, more particularly from the groups of corresponding bacterial and/or mycorrhiza strains.

## Revendications

1. Procédé d'augmentation de la croissance de la flore microbienne du sol et de la stimulation de la décomposition microbienne d'un sol chargé avec une substance organique (substrat), ainsi que donc un renforcement durable lié à la santé des plantes et à la stimulation d'une croissance saine des plantes dans ce substrat,
**caracterise par**
l'introduction de préparations aqueuses contenant
(a) des agents mouillant écologiquement tolérables de type H/E huile dans eau, ensemble avec
(b) des composés organiques ayant un reste hydrocarboné lipophile saturé et/ou insaturé en oléfine avec une structure de matière grasse, dégradables aussi bien par voie aérobie que par voie anaérobie, en tant que fournisseurs supplémentaires de carbone pour la croissance de la flore microbienne,
lié à une introduction simultanée et/ou déplacée temporairement de
(c) composés du phosphore et de l'azote ayant des résidus au moins partiellement lipophiles ainsi que si besoin des matières supports comportant d'autres macro- et/ou micro-substances nutritives pour la croissance des plantes.

2. Procédé selon la revendication 1,
**caractérisé en ce que**
comme constituant (a) on utilise des tensioactifs dégradables biologiquement de caractère non-ionique au moins prédominant, qui en outre sont de préférence en partie au moins prédominante des matières d'origine naturelle et montrent avec cela un indice HLB préféré dans la gamme de 10 à 18.

3. Procédé selon les revendications 1 et 2,
**caractérisé en ce que**
comme constituant (a) on utilise au moins en partie de préférence prépondérante des composés d'alkyl(oligo)glucoside (composés APG), dont les résidus alkyles se dérivent au moins principalement d'alcools gras à chaîne linéaire.

4. Procédé selon les revendications 1 à 3,
**caractérisé en ce que**
sont utilisés les composés APG de glucose et en particulier des alcools gras à base de matière naturelle ayant 6 atomes de carbone, de préférence avec 8 à 24 atomes de carbone et d'indice DP dans la gamme de 1,2 à 5.

5. Procédé selon les revendications 1 à 4,
**caractérisé en ce que**
comme constituant (b) on utilise des composés organiques solubles dans l'huile mais biologiquement tolérables, ayant des résidus d'hydrocarbure aliphatiques et/ou oléfiniquement msaturés, de préférence au moins principalement à chaîne linéaire ayant au moins 6 atomes de carbone et en particulier au moins 8 atomes de carbone.

6. Procédé selon les revendications 1 à 5,
**caractérisé en ce que**
sont utilisés les constituants (b) qui sont fonctionnalisés au moins en partie avec l'oxygène comme hétéroatome, de préférence par l'introduction d'alcool gras et/ou d'acide gras et/ou leurs dérivés, comme leurs esters et/ou esters partiels, éthers et/ou amides.

7. Procédé selon les revendications 1 à 6,
**caractérisé en ce que**
les constituants (b) sont au moins principalement à base de matière naturelle.

8. Procédé selon les revendications 1 à 7,
**caractérisé en ce que**
les constituants (b) présentent au moins en partie des points d'écoulement égaux ou inférieurs à 25-30°C et en particulier égaux ou inférieurs à 10-15°C.

9. Procédé selon les revendications 1 à 8,
**caractérisé en ce que**
comme constituants (b) sont utilisés des alcools gras en C₁₂₋₂₄ insaturés en oléfine d'origine naturelle, en particulier au moins principalement des alcools gras en C_{16/18}, ayant un degré élevé de double liaison de type oléfine et l'intervalle de solidification égal ou inférieur à 20°C, de préférence égal/inférieur à 10-15°C, et/ou d'ester partiel d'acide gras comme le monooléate de glycerine, de préférence des mélanges de ces constituants (b).

10. Procédé selon les revendications 1 à 9,
**caractérisé en ce que**
les alcools gras et esters partiels sont utilisés dans des proportions de mélange de 1 :1 à 1 :10, de préférence de 1 :1 à 1 :5 et en particulier de 1 :1 à 1 :3 parties en poids.

11. Procédé selon les revendications 1 à 10,
**caractérisé en ce que**
comme composés ayant des résidus au moins en partie lipophiles dans le constituant (c) sont utilisés des composés du phosphore et/ou d'azote solubles dans l'huile.

12. Procédé selon les revendications 1 à 11,
**caractérisé en ce que**
comme composés ayant des résidus au moins en partie lipophiles dans le constituant (c) sont utilisés des composés de phosphore et/ou d'azote solubles dans l'huile,

13. Procédé selon les revendications 1 à 12,
**caractérisé en ce que**
comme constituants (c) sont utilisés la lécithine, l'hydrolysat de lécithine et/ou la lécithine modifiée chimiquement, de préférence en mélange avec d'autres macro-substances nutritives comprenant de l'azote, en particulier l'urée et/ou un dérivé d'urée comme autre constituant comprenant de l'azote.

14. Procédé selon les revendications 1 à 13,
**caracterisé en ce que**
les constituants (c) sont introduits sous la forme de préparations aqueuses et de préférence aussi ici par une co-utilisation d'agents mouillants écologiquement tolérables dans le substrat du sol.

15. Procédé selon les revendications 1 à 14,
**caractérisé en ce que**
les constituants (b) et, avec la biodégradation des constituants (a), les mélanges de constituants (b) et (a) sont utilisés dans de tels rapports de quantité vis-à-vis des constituants (c), que des rapports de poids de C :P sont garantis au moins dans 1a gamme de 10 à 50 :1, de préférence au moins dans la gamme de 80 à 150 :1, mais peuvent atteindre des valeurs jusqu'à 500 :1 et au dessus.

16. Procédé selon les revendications 1 à 15,
**caractérisé en ce que**
dans l'application pratique sur la coordination des quantités des constituants (a), (b) et (c ) introduits dans le substrat, des rapports de poids de P :N :C sont ajustés dans la gamme au moins d'environ 1 :10 :10 à 1 :10 :100.

17. Procédé selon les revendications 1 à 16,
**caractérisé en ce que**
si on utilise des phospholipides en tant que fournisseurs de phosphore, dans le constituant (c), on les emploie dans des quantités de 0,01 à 10 g/m² de superficie du substrat, de préférence dans des quantités de 0,1 à 5 g/m² de superficie de substrat, et les constituants (a)/(b) dans des quantités adaptées au rapport molaire souhaité C :P.

18. Concentrés aqueux diluablcs dans l'eau, de matières de support pour l'augmentation de la croissance de la flore microbienne du sol stimulant la croissance des plantes, contenant :
- des composés de phosphore et/ou d'azote au moins en partie solubles dans l'huile et de si besoin d'autres matières support contenant des macro- et/ou micro- substances nutritives pour la croissance des plantes,
- ensemble avec des agents mouillants de type H/E comptatibles biologiquement et en particulier dégradables microbiologiquement,
**caractérisés en ce qu'**
ils contiennent en plus
- des fournisseurs de carbone finement divisés émulsionnables et/ou dispersibles pour la croissance de la flore microbienne, de la classe des composés organiques dégradables par voie aérobie comme aussi anaérobie et comprenant des résidus d'hydrocarbures saturés et/ou insaturés en oléfine, présentant une structure de matière grasse.

19. Mélanges pluri-constituants selon la revendication 18,
**caractérisé en ce que**
comme fournisseurs de carbone ils contiennent des composés organiques fonctionnalisés avec l'oxygène présentant une structure au moins principalement de matière grasse, qui sont répartis au moins à une des classes de matières suivantes : alcool gras et/ou acide gras et/ou leurs sels ainsi que leurs dérivés, comme l'ester et/ou l'ester partiel avec des alcools, éther et/ou amide mono et/ou polyvalents.

20. Mélanges pluri-constituants selon les revendications 18 et 19.
**caractérisé en ce qu'**
ils contiennent des fournisseurs de carbone, en quantité relative avec des constituants contenant du P, telle que des rapports de C :P en dégradation biologique, pour des quantités pondérables et concernant les fournisseurs de carbone par rapport aux parties organiques ayant une structure de matière grasse, sont garantis d'au moins 10 à 50 :1, de préférence d'au moins 80 à 100 :1.

21. Mélanges pluri-constituants selon les revendications 18 à 20,
**caractérisé en ce que**
comme agents mouillants de type H/E ils contiennent des composés APG du glucose et en particulier des alcools gras à base de matière naturelle ayant au moins 6 atomes de carbone, de préférence 8 à 24 atomes de carbone, d'indice DP dans la gamme de 1 à 5, en particulier dans la gamme de 1,2 à 3, et d'indice HLB dans la gamme de 10 à 18.

22. Mélanges pluri-constituants selon les revendications 18 à 21,
**caractérisé en ce qu'**
ils contiennent des constituants de mélange présentant du P et N, qui sont au moins en partie sous la forme de mélanges organiques lipophiles, de préférence par introduction de phospholipides correspondants.

23. Mélanges pluri-constituants selon les revendications 18 à 22,
**caractérisé en ce que**
ils contiennent en tant que phospholipide une lécithine, des hydrolysats de lécithine et/ou la lécithine modifiée chimiquement.

24. Mélanges pluri-constituants selon les revendications 18 à 23,
**caractérisé en ce que**
ils contiennent en plus de l'urée et/ou des dérivés d'urée en tant que source de N.

25. Utilisation du mode de travail d'une augmentation de croissance saine de la flore microbienne du sol et liée à cela de la stimulation et du développement de la faune du sol par l'introduction en particulier de préparations aqueuses de mélanges de P et/ou de N solubles au moins en partie dans l'huile et de si besoin d'autres matières supports contenant des macro- ct/ou micro- substances nutritives pour la croissance des plantes, avec en même temps et/ou de maniére différée l'introduction supplémentaire de fournisseurs de carbone pour la croissance de la flore microbienne à base de composés organiques dégradables non seulement par aérobie mais aussi par anaérobie, contenant des résidus d'hydrocarbure lipophiles saturés et/ou insaturés en oléfine ayant une structure de matière grasse, pour un traitement du sol avec un effet accélérateur de rouissement (minéralisation), avec un effet améliorant du sol (amélioration de l'aération du sol, élimination de la zone du sol anaérobie) et en particulier avec des fortifiants de plantes, ayant de préférence des effets d'engrais à libération prolongée, comme moyen de revitalisation.

26. Utilisation selon la revendication 25,
**caractérisée en ce qu'**
au moins les fournisseurs de carbone pour la croissance de la microflore sont présentés ensemble avec les agents mouillants dégradables microbiologiquement de type H/E, et sont insérés dans la terre sous forme de solution aqueuse diluée, d'émulsion et/ou de suspension.

27. Utilisation selon les revendications 25 et 26 pour l'alimentation et l'enrichissement dans la zone de la rhizosphère. en particulier de fournisseurs de carbone dégradables par aérobie et donc en contact direct avec la surface de la racine des plantes.

28. Utilisation selon les revendications 25 à 27.
**caractérisée par**
la co-utilisation des structures de départ des micro-organismes stimulant la croissance saine des plantes, en particulier des souches de bactéries et/ou de mykorrhiza correspondantes sur les zones.
